# FASCICULE DE BREVET EUROPEEN

(11) **EP 1 715 945 B1**
(45) Date de publication et mention de la délivrance du brevet: **23.12.2015**
(21) Numéro de dépôt: 05727231.2
(22) Date de dépôt: 27.01.2005
(51) Int. Cl.: B01F 17/00, C08F 2/32

(54) **AGENTS EPAISSISSANTS**
VERDICKUNGSMITTEL
THICKENING AGENTS

(30) Priorité: 17.02.2004 FR 0450287
(43) Date de publication de la demande: 02.11.2006
(73) Titulaire: S.P.C.M. SA, 42160 Andrézieux Bouthéon (FR)
(72) Inventeur: VILLARD, Emmanuel, F-42230 SAINT CHRISTO EN JAREZ (FR); CHAMPAGNON, Lionel, F-42600 MAGNEUX HAUTE RIVE (FR); JEHN-RENDU, Fabienne, F-42170 SAINT JUST SAINT RAMBERT (FR)
(74) Mandataire: Cabinet Laurent & Charras
(86) Numéro de dépôt international: PCT/FR2005/050047
(87) Numéro de publication internationale: WO 2005/079965

(56) Documents cités:
- EP-A- 0 186 361
- WO-A-94/01073
- US-A- 5 216 070
- DATABASE WPI Section Ch, Week 200306 Derwent Publications Ltd., London, GB; Class A14, AN 1995-136849 XP002299824 & JP 03 361854 B2 (NIPPON MEKTRON KK) 7 janvier 2003 (2003-01-07) cité dans la demande

## Description

La présente invention concerne le domaine des polymères synthétiques, obtenus majoritairement à partir de monomères solubles dans l'eau, ou de mélanges de tels monomères, leur procédé de préparation et leurs applications en tant qu'épaississant et/ou émulsionnant et/ou stabilisant.

Plus particulièrement, l'invention concerne une émulsion inverse à base de polymères réticulés à motifs ioniques, anioniques et/ou cationiques, comprenant une phase huile de type silicone, une phase aqueuse, au moins un émulsifiant à base silicone, et au moins un agent émulsifiant de type huile dans eau (H/E), ladite émulsion comprenant de 10 à 80% en poids d'au moins un polymère réticulé anionique, cationique ou amphotère.

Par polymères réticulés, on entend dans la présente invention, des polymères résultant de l'utilisation, lors de leur polymérisation, d'un agent réticulant ou ramifiant.

Le terme de silicone s'applique à une vaste famille de produits pouvant avoir des propriétés très différentes les uns des autres. D'une façon générale, les silicones sont des matières synthétiques composées des éléments silicium et oxygène combinés à des groupes organiques. Suivant la nature du groupe organique et des conditions de polymérisation, les silicones peuvent être des substances de différentes natures (fluides, résines...).

On connaît dans l'art antérieur :
- Le brevet EP 186361 qui présente un épaississant sous forme d'une émulsion inverse. Dans la description de l'invention, il est précisé qu'au moins une partie de la phase huile de l'émulsion E/H peut être une huile silicone.
   L'émulsion inverse contient en outre un émulsifiant du type huile dans eau listé page 9, lignes 10-15, l'émulsion est exempte d'agent d'inversion et d'un agent émulsifiant du type eau dans silicone.
- Le brevet WO 02/44228 relatif à la préparation de polymères sous forme d'une dispersion liquide (pas ou peu de phase aqueuse : eau < 3%).
- Le document US-A-5216070 décrivant une émulsion E/H comportant un émulsifiant du type silicone incorporé dans une phase huileuse exempte de silicone
- Le document JP-03 361854 divulguant une microémulsion E/H et non pas une émulsion, qui par définition, présente une taille de particules supérieure. En outre, elle ne contient pas d'agent d'inversion. EP 992 280 divulgue une émulsion eau dans silicone.

Un problème technique extrêmement important pour les polymères synthétiques ayant pour rôle d'épaissir et/ou d'émulsionner et/ou de stabiliser des compositions aqueuses ou des émulsions consiste à avoir une forte compatibilité avec les autres composants utilisés dans les compositions finales afin que la stabilité de celles-ci soit optimale.

La fabrication des silicones est assez récente puisqu'elle ne remonte qu'à une cinquantaine d'années. Toutefois, l'évolution des recherches et des connaissances a permis la synthèse de nouvelles combinaisons de ces polymères, diversifiant ainsi encore plus leurs potentialités.

Les produits à base silicone disponibles aujourd'hui sur le marché ont des propriétés très variées et sont utilisées par des secteurs d'activités très différents les uns des autres. L'utilisation croissante des composés siliconés et donc des volumes synthétisés a significativement diminué les prix de ces produits. D'un point de vue économique, il est maintenant possible d'envisager de les incorporer dans des projets, même ceux visant à aboutir à des produits dont le prix de revient est un paramètre essentiel.

De façon induite, une forte demande existe pour épaissir et stabiliser des compositions à base de silicone, dont la solution n'a pas été apportée de manière satisfaisante par l'art antérieur.

Etant donné que les silicones sont à la fois des composés inorganiques et organiques, leurs propriétés chimiques reflètent ce double caractère. Pour l'homme de métier, cette dualité va entraîner lors de la préparation d'émulsions de polymères aussi bien des problèmes d'émulsification que des problèmes de stabilité du mélange réactionnel et de l'émulsion après polymérisation.

Ainsi, à ce jour, aucun polymère synthétique de type épaississant n'a été décrit ou n'est proposé sous la forme d'une émulsion eau dans silicone.

Plus particulièrement, la seule solution efficace qui semble avoir été trouvée consiste a utiliser une dispersion, telle que présentée dans le brevet WO 02/44228, qui a pour avantage de ne pas avoir de phase aqueuse, ce qui supprime de fait les problèmes de stabilité qui lui sont liés. Il est à noter que, même si ce brevet propose une solution technique potentielle aux problèmes rencontrés par l'homme de métier, celle-ci manque cruellement de description, il n'y a en particulier aucun exemple présenté.

Le problème technique correspondant à l'invention est donc de proposer une émulsion eau dans silicone présentant une capacité épaississante et/ou émulsionnante et/ou stabilisante.

On entend, dans toute la présente demande par « polymère ou copolymère réticulé ou ramifié », un polymère ou copolymère réticulé ou ramifié obtenu par polymérisation sous forme d'émulsion inverse, bien connue de l'homme de métier.

Par "agent d'inversion", on désigne un surfactant (ou un mélange de surfactants) qui a pour effet de permettre l'inversion de phase de l'émulsion et la dispersion du polymère dans le milieu aqueux lors de son utilisation. Celui-ci possède généralement une valeur HLB (balance hydrophile/lipophile) suffisamment élevée pour obtenir des émulsions huile dans eau. En général, la valeur de HLB moyenne doit être supérieure à 8. Parmi les principales familles "d'agents d'inversion", on peut citer : les alcools gras éthoxylés, les esters d'acide gras - du sorbitan - de polyéthylène glycols - de glycérol, les alkyl polyglucosides... Certains composés siliconés tels que les diméthicone copolyols peuvent également être utilisés.

Selon la présente invention, il a été mis au point une une émulsion inverse "eau dans silicone" à base de polymères permettant d'épaissir, émulsionner et/ou stabiliser des compositions aqueuses ou des émulsions aussi bien à pH acide qu'à pH basique tout en apportant à la composition finale obtenue des caractéristiques physiques optimales, notamment si elle contient aussi des composés siliconés.

L'émulsion de l'invention est susceptible être obtenue par polymérisation (ou respectivement copolymérisation, ensemble dans tout le texte et les revendications: "polymérisation") d'au moins un monomère ionique et éventuellement d'autres monomères non ioniques, en présence d'au moins un agent réticulant et éventuellement d'au moins un agent de transfert et présente :
- une phase continue composée d'au moins une huile de type silicone,
- au moins 2 surfactants, dont au moins un est un émulsifiant siliconé de type eau dans silicone et au moins un est un agent d'inversion.

L'homme de métier saura apprécier, à partir de ses connaissances propres ou à l'aide d'essais de routine, le degré d'agent de transfert et les conditions de polymérisation à utiliser pour obtenir une émulsion finale présentant une viscosité intrinsèque telle que requise.

Dans un mode de réalisation avantageux, la phase continue est composée à 100% en poids d'au moins une huile du type silicone.

De plus, il est également possible de concentrer l'émulsion par toutes les techniques connues, comme par exemple par distillation azéotropique.

Selon un mode de réalisation préféré, le copolymère est obtenu à partir de :
- 10 à 100% molaire d'au moins un monomère possédant une charge ionique
- 0 à 90% molaire d'au moins un monomère possédant une charge neutre
- la concentration en matière active, lors de la polymérisation est de préférence comprise entre 20 et 50%
- et le taux de réticulation est supérieure à 10 ppm, de préférence supérieur à 50 ppm et de préférence supérieur à 200 ppm (en considérant le MBA) par rapport au polymère ou une réticulation équivalente avec un agent réticulant d'efficacité différente.

On trouvera ci-dessous une liste non limitative des monomères pouvant être utilisés :
a/ les monomères ioniques :
   - les monomères cationiques : de type dialkylaminoalkyl (meth)acrylate, dialkylaminoalkyl (meth)acrylamide, diallylamine, methyldiallylamine et leurs sels d'ammonium quaternaire ou d'acides...
   - les monomères anioniques: possédant une fonction carboxylique (ex : acide acrylique, acide methacrylique, et leurs sels...), les monomères possédant une fonction acide sulfonique (ex : acide 2-acrylamido-2-méthylpropane sulfonique (AMPS) et leurs sels...)...
b/ les monomères non ioniques : acrylamide, methacrylamide, N-vinyl pyrrolidone, vinylacetate, alcool vinylique, esters acrylate, alcool allylique...

Il est important de noter que, en association avec ces monomères, il est également possible d'utiliser au moins un monomère insoluble dans l'eau tel que les monomères acryliques, allyliques ou vinyliques comportant un groupement hydrophobe.

On trouvera ci-dessous une liste non limitative des réticulants : methylene bisacrylamide (MBA), l'ethylene glycol di-acrylate, le polyethylene glycol dimethacrylate, le diacrylamide, le cyanomethylacrylate, le vinyloxyethylacrylate ou methacrylate et le formaldehyde, le glyoxal, les composés de type glycidyléther comme l'éthylèneglycol diglycidyléther, ou des époxy ou tout autre moyen bien connu de l'homme de métier permettant la réticulation.

Dans la suite de la description et dans les revendications, par « agent émulsifiant siliconé de type eau dans silicone », on désigne un surfactant à base de silicone contenant une partie soluble à l'eau (hydrophile) et une partie soluble dans les fluides siliconés (siliphile). Il représente en pratique entre 5 et 20 % en poids par rapport à l'émulsion, de préférence entre 7 et 15 %.

Dans un mode de réalisation avantageux, l'agent émulsifiant siliconé de type eau dans silicone est non-ionique.

En pratique, l'agent émulsifiant siliconé de type eau dans silicone est choisi dans le groupe comprenant le diméthicone copolyol, les silicones alkanolamides, les silicones esters, les silicones glycosides, etc....

On trouvera ci-dessous une liste non limitative des agents de transfert : alcool isopropylique, hypophosphite de sodium, mercaptoethanol, etc...En pratique, l'agent de transfert peut être utilisé lors de la polymérisation.

Selon une autre caractéristique, l'agent d'inversion représente entre 2 et 10 % en poids par rapport à la charge (émulsion + inverseur), de préférence entre 2,5 et 6 % en poids.

L'homme de métier saura choisir la meilleure combinaison en fonction de ses connaissances propres et de la présente description, ainsi que des exemples qui vont suivre.

### EXEMPLES

### 1/ Polymérisation

Chacun des polymères décrits ci-après a été obtenu par polymérisation radicalaire sous forme d'émulsion inverse. Une phase aqueuse contenant le(s) monomère(s) est finement dispersée dans une huile de type silicone contenant en particulier au moins un agent émulsifiant à base silicone. Le mélange est alors dégazé et l'initiation de la polymérisation est réalisée de manière conventionnelle.

| **EXEMPLES** | **ESSAI 1** | | **ESSAI 2** | | **ESSAI 3** | | **ESSAI 4** | | **ESSAI 5** | |
|---|---|---|---|---|---|---|---|---|---|---|
| **PHASE AQUEUSE** | | | | | | | | | | |
| Monomère(s) | Acide acrylique Sel de sodium | 100%mol | Acide acrylique Sel de sodium | 100%mol | Acide acrylique Sel de sodium | 100%mol | Acide acrylique Sel de sodium | 100%mol | Acide acrylique Sel de sodium | 100%mol |
| Agent réticulant | MBA (12) | 550ppm (1) | MBA (12) | 550ppm (1) | MBA (12) | 550ppm (1) | MBA (12) | 550ppm (1) | MBA (12) | 550ppm (1) |

| PHASE ORGANIQUE | | | | | | | | | | |
|---|---|---|---|---|---|---|---|---|---|---|
| Huile(s) | CycloPenta Siloxane (8) | | | | PDMS (2) 10 est | | | | | |
| Emulsifiant(s) ou stabilisant(s) | Mono oleate de sorbitan | 2% (3) | Mono oleate de sorbitan | 4% (3) | Mono oleate de sorbitan | 2% (3) | Mono oleate de sorbitan | 7% (3) | Mono oleate de sorbitan | 10% (3) |
| Rapport phase aqueuse/phase organique | 70/30 | | | | | | | | | |
| Concentration en monomère (s) pendant la polymérisation % (3): | 26 | | | | | | | | | |
| Observations | COMPOSITIONS INSTABLES : | | | | | | | | | |
| | déphasage des émulsions inverses avant même la polymérisation | | | | | | | | | |

| | **ESSAI A** | | **ESSAI B** | | **ESSAI C** | | **ESSAI D** | | **ESSAI E** | |
|---|---|---|---|---|---|---|---|---|---|---|
| **PHASE AQUEUSE** | | | | | | | | | | |
| Monomère(s) | Acide acrylique Sel de sodium | 100%mol | Acide acrylique Sel de sodium | 100%mol | Acide acrylique Sel de sodium | 100%mol | Acide acrylique Sel de sodium | 100%mol | Acide acrylique Sel de sodium | 100%mol |
| Agent réticulant | MBA (12) | 550ppm (1) | MBA (12) | 550ppm (1) | MBA (12) | 550ppm (1) | MBA (12) | 30ppm (1) | MBA (12) | SSOppm (1) |

| PHASE ORGANIQUE | | | | | | | | | | |
|---|---|---|---|---|---|---|---|---|---|---|
| Huile(s) | PDMS (2) 5 cst | | | | PDMS (2) 10 cst | | PDMS (2) 20 cst | | CycloPenta Siloxane (8) | |
| Emulsifiant(s) ou stabilisant(s) | Emulsifi ant silicone N°1 (4) | 10% (3) | Emulsifiant silicone N°2 (5) | 10% (3) | Emulsifiant silicone N°1 (4) | 10% (3) | Emulsifiant silicone N°1 (4) | 10% (3) | Emulsifiant silicone N°1 (4) | 10% (3) |
| Rapport phase aqueuse/phase organique | 65 / 35 | | | | | | | | | |
| Concentration en monomère (s) pendant la polymérisation % (3) | 26 | | | | | | | | | |
| Observations | Emulsions inverses stables | | | | | | | | | |
| Viscosité d'une solution aqueuse à 1% de polymère (6) | 29000cps | | 31500cps | | 27000cps | | 2000cps | | 30500cps | |

| | **ESSAI F** | | **ESSAI G** | | **ESSAI H** | | **ESSAI I** | | **ESSAI J** | |
|---|---|---|---|---|---|---|---|---|---|---|
| **PHASE AQUEUSE** | | | | | | | | | | |
| Monomère(s) | Acide acrylique Sel de sodium | 100%mol | Acide acrylique AMPS (9) Sel de sodium | 30%mol 70%mol | MADAME quaternisé (10) AM | 80%mol 20%mol | APTAC (11) | 100%mol | APTAC (11) acide acrylique sel de sodium | 50%mol |
| Agent réticulant | MBA (12) | 800ppm (1) | MBA (12) | 550ppm (1) | MBA (12) | 300ppm (1) | MBA (12) | 600ppm (1) | MBA (12) | 1000ppm |

| PHASE ORGANIQUE | | | | | | | | | | |
|---|---|---|---|---|---|---|---|---|---|---|
| Huile(s) | PDMS (2) 5 est Isopar J (7) | | PDMS (2) 5 est | | | | | | | |
| Emulsifiant(s) ou stabilisant(s) | Emulsifi ant silicone N°1 (4) | 10% (3) | Emulsifiant silicone N°1 (4) | 10% (3) | Emulsifiant silicone N°1 (4) | 10% (3) | Emulsifiant silicone N°1 (4) | 7% (3) | Emulsifiant silicone N°1 (4) | 4% (3) |
| Rapport phase aqueuse/phase organique | 70/30 | | | | | | | | | |
| Concentration en monomère (s) pendant la polymérisation % (3) | 26 | | 30 | | 40 | | 40 | | 40 | |
| Observations | Emulsions inverses STABLES | | | | | | | | | |
| Viscosité d'une solution aqueuse à 1% de polymère (6) | 40000 cps | | 20000cps | | 4000cps | | 5000cps | | 6000 cps | |

Mesure des viscosités : les viscosités sont mesurées avec un brookfield RVT - 20RPM. Le pH est ajusté avec une solution d'acide citrique.

Pour chacun des essais A à J, un surfactant de type huile dans eau (par exemple le Trideceth 6) a été ajouté à des concentrations d'au moins 2% en fin de process. Celui-ci a eu pour effet de permettre l'inversion de phase et la dispersion du polymère dans le milieu aqueux lors de son utilisation.
(1) ppm (parties par millions) / monomères totaux
(2) PDMS: polydiméthylsiloxane - Série DC200 de la société DOW CORNING
(3) % / masse d'émulsion inverse
(4) Tensio actif silicone non-ionique N°1 : DC5225C de la société DOW CORNING - Nom INCI: cyclopentasiloxane and PEG/PPG-18/18 Dimethicone
(5) Tensio actif silicone non-ionique N°2: DC9011 de la société DOW CORNING - Nom INCI : cyclopentasiloxane and PEG-12 dimethicone crosspolymer
(6) Mesure avec un brookfield RVT 20 RPM - 25°C
(7) Isopar J : Hydrocarbure isoparaffine
(8) Cyclopentasiloxane - DC245 de la société DOW CORNING
(9) AMPS : Acide de 2 acrylamide 2 méthyl propane sulfonique
(10) MADAME : Méthacrylate de diméthyl amino éthyle
(11) APTAC : Chlorure de triméthyl ammonium propyl acrylamide
(12) MBA : Methylene bis acrylamide

### 2/ Conclusion

Il apparaît à la vue des résultats ci-dessus que l'obtention d'un polymère sous forme d'émulsion inverse "eau dans silicone" nécessite des développements spécifiques.

Celui-ci ne peut, en effet, être obtenu à l'aide de conditions standard de polymérisation comme le démontrent les résultats des essais 1 à 5. Ceci explique pourquoi, à ce jour, aucun polymère synthétique de type épaississant n'a été décrit ou n'est proposé sous la forme d'une émulsion eau dans silicone.

Selon la présente invention, il a été découvert que, de manière surprenante, grâce à un choix approprié des conditions de polymérisation (au moins 2 surfactants, dont au moins un est un émulsifiant non standard à base silicone et au moins un est un agent d'inversion), une nouvelle famille de polymères permet d'épaissir, émulsionner et/ou stabiliser des compositions aqueuses ou des émulsions, aussi bien à pH acide qu'à pH basique, tout en apportant à la composition finale obtenue une très forte stabilité et des caractéristiques physiques optimales (cf mesures de viscosité), notamment si elles contiennent aussi des composés siliconés (très bonne compatibilité).

Les exemples ci-dessus montrent la diversité des polymères qui peuvent être obtenus par ce nouveau type d'émulsions "eau dans silicone", à la fois pour leurs propriétés épaississantes, émulsionnantes et/ou stabilisantes.

Les polymères en émulsion tels que nous les avons définis peuvent être incorporés à toute température. Ils offrent, également, une grande souplesse quant à l'étape d'incorporation.

L'invention concerne l'utilisation de cette famille de (co)polymères comme agent épaississant et/ou émulsionnant et/ou stabilisant.

L'homme de métier saura adapter la présente invention à des options ou variantes non expressément décrites, sans sortir du cadre de la présente invention.

## Revendications

1. Emulsion inverse "eau dans silicone" susceptible d'être obtenue par polymérisation ou copolymérisation d'au moins un monomère ionique, anionique et/ou cationique, et éventuellement au moins un monomère non-ionique, en présence d'un agent réticulant et éventuellement d'un agent de transfert, et présentant :
- une phase continue composée à 100% en poids d'une huile de type silicone,
- et au moins 2 surfactants, dont au moins un est un émulsifiant siliconé non ionique de type eau dans silicone et au moins un est un agent d'inversion.

2. Emulsion selon la revendication 1, **caractérisée en ce que** le monomère ionique de type cationique est choisi dans le groupe comprenant le dialkylaminoalkyl (meth)acrylate, le dialkylaminoalkyl (meth)acrylamide, le diallylamine, le methyldiallylamine et leurs sels d'ammonium quaternaire ou d'acides.

3. Emulsion selon la revendication 1, **caractérisée en ce que** le monomère ionique de type anionique est choisi dans le groupe comprenant les monomères possédant une fonction carboxylique : acide acrylique, acide methacrylique, et leurs sels, les monomères possédant une fonction acide sulfonique : acide 2-acrylamido-2-méthylpropane sulfonique et leurs sels.

4. Emulsion selon la revendication 1, **caractérisée en ce que** le ou les monomères non-ioniques sont choisis dans le groupe comprenant l'acrylamide, le methacrylamide, la N-vinyl pyrrolidone, le vinylacetate, l'alcool vinylique, les esters d'acrylate et/ou l'alcool allylique.

5. Emulsion selon la revendication 4, **caractérisée en ce que** le monomère non ionique est la N-vinyl pyrrolidone.

6. Emulsion selon l'une quelconque des revendications 1 à 5, **caractérisée en ce que** l'agent réticulant est choisi dans le groupe comprenant le methylene bisacrylamide, l'ethylene glycol di-acrylate, le polyethylene glycol dimethacrylate, le diacrylamide, le cyanomethylacrylate, le vinyloxyethyl(meth)acrylate, le formaldehyde, le glyoxal, les composés de type glycidyléther ou époxy.

7. Emulsion selon l'une quelconque des revendications 1 à 6, **caractérisée en ce que** l'agent de transfert est choisi dans le groupe comprenant l'alcool isopropylique, l'hypophosphite de sodium, ou le mercaptoethanol.

8. Emulsion selon l'une quelconque des revendications 1 à 7, **caractérisée en ce que** la concentration en matière active lors de la polymérisation est comprise entre 20 à 50% en poids.

9. Emulsion selon l'une quelconque des revendications 1 à 8, **caractérisée en ce que** l'agent d'inversion possède - soit une valeur HLB moyenne supérieure à 8 et est choisi dans le groupe comprenant les alcools gras éthoxylés, les esters d'acide gras - du sorbitan - de polyéthylène glycols - de glycérol, les alkyl polyglycosides - soit est de type siliconé tels que les diméthicone copolyols.

10. Emulsion selon l'une quelconque des revendications 1 à 9, **caractérisée en ce que** le taux de réticulation du polymère, sur la base du méthylène bis acrylamide supérieure à 10 ppm par rapport au polymère, de préférence supérieure à 50 ppm, et de préférence supérieure à 200 ppm.

11. Emulsion selon l'une des revendications 1 à 10, **caractérisée en ce que** la polymérisation ou copolymérisation est effectuée en présence d'au moins un monomère insoluble dans l'eau tel que les monomères acryliques, allyliques ou vinyliques comportant un groupement hydrophobe.

12. Emulsion selon l'une des revendications 1 à 11, **caractérisée en ce que** l'agent émulsifiant siliconé de type eau dans silicone est choisi dans le groupe comprenant le diméthicone copolyol, les silicones alkanolamides, les silicones esters, les silicones glycosides.

## Patentansprüche

1. "Wasser-in-Silikon"-Umkehremulsion, die durch Polymerisierung oder Copolymerisierung mindestens eines ionischen, anionischen und/oder kationischen Monomers und gegebenenfalls mindestens eines nichtionischen Monomers im Beisein eines Vernetzungsmittels und gegebenenfalls eines Übertragungsmittels erhalten werden kann und aufweist:
- eine kontinuierliche Phase, die sich zu 100 Gew.-% aus einem Öl des Typs Silikon zusammensetzt,
- und mindestens zwei Tenside, wobei zumindest eines ein nichtionischer silikon-haltiger Emulgator des Typs Wasser in Silikon und das andere ein Umkehrmittel ist.

2. Emulsion nach Anspruch 1, **dadurch gekennzeichnet, dass** das ionische Monomer des kationischen Typs aus der Gruppe ausgewählt ist, die Dialkylaminoalkyl(meth)acrylat, Dialkylaminoalkyl(meth)acrylamid, Diallylamin, Methyldiallylamin und deren quaternären Ammoniumsalze oder Säuresalze umfasst.

3. Emulsion nach Anspruch 1, **dadurch gekennzeichnet, dass** das ionische Monomer des anionischen Typs aus der Gruppe ausgewählt ist, die Monomere umfasst, die eine Carboxylfunktion besitzen: Acrylsäure, Methacrylsäure und deren Salze, Monomere, die eine Sulfonsäurefunktion besitzen: 2-Acrylamid-2-methylpropansufonsäure und deren Salze.

4. Emulsion nach Anspruch 1, **dadurch gekennzeichnet, dass** das oder die nichtionische/n Monomer/e aus der Gruppe ausgewählt ist/sind, die Acrylamid, Methacrylamid, N-Vinylpyrrolidon, Vinylacetat, Vinylalkohol, Acrylatester und/oder Allylalkohol umfasst.

5. Emulsion nach Anspruch 4, **dadurch gekennzeichnet, dass** das nichtionische Monomer N-Vinylpyrrolidon ist.

6. Emulsion nach einem der Ansprüche 1 bis 5, **dadurch gekennzeichnet, dass** das Vernetzungsmittel aus der Gruppe ausgewählt ist, die Methylenbisacrylamid, Ethylenglycoldiacrylat, Polyethylenglycoldimethacrylat, Diacrylamid, Cyanomethacrylat, Vinyloxyethyl(meth)acrylat, Formaldehyd, Glyoxal, Verbindungen des Typs Glycidylether oder Epoxy umfasst.

7. Emulsion nach einem der Ansprüche 1 bis 6, **dadurch gekennzeichnet, dass** das Übertragungsmittel aus der Gruppe ausgewählt ist, die Isopropylalkohol, Natriumhypophosphit oder Mercaptoethanol umfasst.

8. Emulsion nach einem der Ansprüche 1 bis 7, **dadurch gekennzeichnet, dass** die Konzentration an aktivem Stoff bei der Polymerisierung 20 bis 50 Gew.-% beträgt.

9. Emulsion nach einem der Ansprüche 1 bis 8, **dadurch gekennzeichnet, dass** das Umkehrmittel - entweder einen mittleren HLB-Wert von über 8 besitzt und aus der Gruppe ausgewählt ist, die Ethoxyfettsäuren, Fettsäureester von - Sorbitan - Polyethylenglykolen - Glycerol, Alkylpolyglycoside umfasst - oder vom Silikontyp ist wie etwa Dimethiconcopolyole.

10. Emulsion nach einem der Ansprüche 1 bis 9, **dadurch gekennzeichnet, dass** der Vernetzungsgrad des Polymers auf der Grundlage von Methylenbisacrylamid höher als 10 ppm in Bezug auf das Polymer ist, vorzugsweise höher als 50 ppm ist, und vorzugsweise höher als 200 ppm ist.

11. Emulsion nach einem der Ansprüche 1 bis 10, **dadurch gekennzeichnet, dass** die Polymerisierung oder Copolymerisierung im Beisein mindestens eines wasserunlöslichen Monomers wie etwa Acryl-, Allyl- oder Vinylmonomeren erfolgt, die eine hydrophobe Gruppe umfassen.

12. Emulsion nach einem der Ansprüche 1 bis 11, **dadurch gekennzeichnet, dass** der silikonhaltige Emulgator des Typs Wasser in Silikon aus der Gruppe ausgewählt ist, die Dimethiconcopolyol, Silikon-Alkanolamide, Silikon-Ester, Silikon-Glycoside umfasst.

## Claims

1. A "water-in-silicone" inverse emulsion obtainable by polymerisation or copolymerisation of at least one ionic, anionic and/or cationic monomer, and possibly at least one non-ionic monomer, in the presence of a cross-linking agent and possibly a transfer agent, and comprising:
- a continuous phase containing at 100% by weight of one silicone-type oil,
- and at least 2 surfactants, at least one of which is a water-in-silicone-type non-ionic silicone emulsifier and at least one is an inversion agent.

2. Emulsion as claimed in claim 1, **characterised in that** the cationic-type ionic monomer is chosen from the group comprising dialkylaminoalkyl (meth)acrylate, dialkylaminoalkyl (meth)acrylamide, diallylamine, methyl diallylamine and their quaternary ammonium or acid salts.

3. Emulsion as claimed in claim 1, **characterised in that** the anionic-type ionic monomer is chosen from the group comprising monomers having a carboxylic function: acrylic acid, methacrylic acid, and their salts, monomers having a sulphonic acid function: 2-acrylamido-2-methyl propane sulphonic acid and their salts.

4. Emulsion as claimed in claim 1, **characterised in that** the non-anionic monomer(s) is (are) chosen from the group comprising acrylamide, methacrylamide, N-vinyl pyrrolidone, vinyl acetate, vinyl alcohol, acrylate esters and/or allyl alcohol.

5. Emulsion as claimed in claim 4, **characterised in that** the non-ionic monomer is N-vinyl pyrrolidone.

6. Emulsion as claimed in any of claims 1 to 5, **characterised in that** the cross-linking agent is chosen from the group comprising methylene bisacrylamide, ethylene glycol diacrylate, polyethylene glycol dimethacrylate, diacrylamide, cyanomethyl acrylate, vinyl oxyethyl (meth)acrylate, formaldehyde, glyoxal, glycidyl ether-type or epoxy-type compounds.

7. Emulsion as claimed in any of claims 1 to 6, **characterised in that** the transfer agent is chosen from the group comprising isopropyl alcohol, sodium hypophosphite or mercaptoethanol.

8. Emulsion as claimed in any of claims 1 to 7, **characterised in that** the concentration of active matter during polymerisation is between 20 and 50% by weight.

9. Emulsion as claimed in any of claims 1 to 8, **characterised in that** the inversion agent either - has a mean HLB value greater than 8 and is chosen from the group comprising fatty alcohol ethoxylates, fatty acid esters - sorbitan - polyethylene glycols - glycerol, alkyl polyglycoside - or is of the silicone type such as dimethicone copolyols.

10. Emulsion as claimed in any of claims 1 to 9, **characterised in that** the cross-linking rate of the polymer, based on methylene bisacrylamide, is greater than 10 ppm in relation to the polymer, preferably greater than 50 ppm, and preferably greater than 200 ppm.

11. Emulsion as claimed in any of claims 1 to 10, **characterised in that** the polymerisation or copolymerisation is undertaken in the presence of at least one non-water-soluble monomer such as acrylic, allylic or vinylic monomers with a hydrophobic group.

12. Emulsion as claimed in any of claims 1 to 11, **characterised in that** the water-in-silicone-type silicone emulsifying agent is chosen from the group comprising dimethicone copolyol, silicone alkanolamides, silicone esters, silicone glycosides.
